# FASCICULE DE BREVET EUROPEEN

(11) **EP 1 205 184 B1**
(45) Date de publication et mention de la délivrance du brevet: **11.03.2009**
(21) Numéro de dépôt: 01402924.3
(22) Date de dépôt: 14.11.2001
(51) Int. Cl.: A61K 38/08, A61K 8/58, A61P 17/02, A61K 8/64, A61Q 19/00

(54) **Composition synergetique à base de méthylsilanol hydroxyproline aspartate et de palmitoyl-pentapeptide-3**
Synerergistische Zusammensetsung aus Methylsilanol Hydroxyprolin Aspartat und Palmitoyl-Pentapeptid
Methylsilanol hydroxyproline aspartate and palmitoyl-pentapeptide-based synergistic composition

(30) Priorité: 14.11.2000 FR 0014642
(43) Date de publication de la demande: 15.05.2002
(73) Titulaire: Thabaut, Simon, 75017 Paris (FR); Becquevort, Michel, 75017 Paris (FR)
(72) Inventeur: Thabaut, Simon, 75017 Paris (FR); Becquevort, Michel, 75017 Paris (FR)
(74) Mandataire: Eidelsberg, Victor Albert

(56) Documents cités:
- WO-A-00/15188
- WO-A-00/62743
- WO-A-98/44904
- US-A- 5 204 105

## Description

L'invention concerne des compositions ayant un effet sur la peau.

La radiothérapie externe, malgré les progrès techniques réalisés, garde des effets secondaires cutanés. Ils peuvent aller d'une simple rougeur à une brûlure du second degré.

Leur survenue dépend de la zone irradiée, de la dose délivrée à la peau, du fractionnement utilisé, du type de rayonnement, de la susceptibilité individuelle des patients.

Les effets cutanés de l'irradiation restent une préoccupation pour les patients et les équipes soignantes du fait de leur fréquence et du fait, aussi, de leur survenue précoce pendant l'irradiation pouvant gêner celle-ci voire, dans certains cas, l'interrompre avec des conséquences potentielles sur l'efficacité du traitement. Ainsi, même s'ils ne sont pas toujours majeurs, ils altèrent la qualité de vie des patients traités, à la fois par leur existence même, source de douleur et d'inconfort mais également par leur impact éventuel sur le déroulement du traitement.

L'invention vise une composition destinée à atténuer les effets néfastes sur la peau induits par les traitements de radiothérapie et ayant, d'une manière générale, des effets favorables sur la peau.

La composition suivant l'invention comprend un palmitoyl-pentapeptide-3 et du méthylsilanol hydroxyproline aspartate.

Le palmitoyl-pentapeptide-3 est de préférence le palmitoyl-Lys-Thr-Thr-Lys-Ser qui a une action puissante sur :
- la stimulation de la synthèse des collagènes I et III et de la fibronectine améliorant ainsi la structure de la matrice tissulaire,
- la stimulation de la synthèse du collagène IV dans la jonction dermo/épidermique où se produit la vascularisation et participe donc au processus de régénération de la peau.

Le méthylsilanol hydroxyproline aspartate, qui se présente sous la forme d'un noyau silanol et de deux composants acides aminés à savoir l'hydroxyproline et l'acide aspartique, restaure les tissus cutanés déstructurés par:
- action cytostimulante : cette action de stimulation du tissu conjonctif accroît sensiblement le nombre de fibres de collagène et agit sur leur orientation qui s'organise parallèlement à la surface de la peau.
- action anti radicalaire : la réorganisation des lipides de la membrane des cellules rend celles-ci moins sensibles à l'attaque des radicaux libres.
- action hydratante sur les tissus superficiels de la peau : hydroxyprolisilane est un agent hydratant biologique : les groupements hydroxyles génèrent une organisation de molécules d'eau autour des molécules silanols constituant ainsi une importante sphère d'hydratation. Cette eau "liée" est en fait disponible pour la peau et représente une réserve pour celle-ci.

Enfin, on constate une action normalisatrice de la perméabilité capillaire superficielle par l'amélioration des échanges au niveau des capillaires veineux, lymphatiques et artériels.

La combinaison de ces deux produits dans la composition suivant l'invention permet de lutter efficacement contre les effets néfastes induits par la radiothérapie en accélérant les effets que l'on obtiendrait en les utilisant séparément.

La composition a aussi les effets suivants :
- apaisement des douleurs et guérison rapide des "coups de soleil"
- apaisement des douleurs et réparation rapide de peaux irritées ou abimées par des actions physiques (épilation) ou chimiques (produits détergents)
- traitement des vergetures (femmes enceintes)
- limitation de l'importance de problèmes cutanés de type acnéique
- amélioration de la répartition cellulaire dans les cicatrices
- sénescence cutanée: amélioration de la texture de la peau, diminution des rides et ridules

Le rapport en poids du palmitoyl-pentapeptide-3 au méthylsilanol hydroxyproline aspartate en solutions est compris en général entre 5/95 et 95/5. La composition contient de préférence de 30 à 50% du pentapeptide pour, de manière correspondante, de 70 à 50% du méthylsilanol hydroxyproline aspartate. On préfère tout particulièrement que le méthylsilanol hydroxyproline aspartate représente 60% de l'ensemble des deux produits.

De préférence, on ajoute également à la composition un polysaccharide de levure qui favorise la phagocytose macrophage et la production de facteurs cytolytiques et cytostatiques. Il a un effet protecteur de la peau et a aussi une influence sur la régénérescence des cellules immunocompétentes (kératinocytes et cellules de Langerhans). Il restaure l'équilibre des fonctions de la peau. Enfin, il est apte à bien absorber l'eau, combiné à une caractéristique filmogène qui en fait un bon hydratant des couches superieures de l'épiderme.

On peut également ajouter à la composition de l'allantoïne et de l'huile d'avocat de manière à faciliter au mieux l'étalement sur les zones cutanées sensibles.

La composition se présente de préférence sur la forme d'une crème contenant de 0,5 à 6% en poids d'une solution de méthylsilanol hydroxyproline aspartate et de 0,5 à 6% en poids d'une solution de palmitoyl-pentapeptide-3. On préfère tout particulièrement que la solution de méthylsilanol hydroxyproline aspartate représente environ 3% du poids de la composition et que celle du palmitoyl-pentapeptide-3 en représente 2%.

L'exemple suivant illustre l'invention.

On mélange 62,4 grammes d'une composition comprenant en poids 6 parties de glycérine, 25 parties d'eau, 25 parties de butylène glycol, 0,15 partie d'un carbomère, 0,15 partie de polysorbate 20 parties et 2 grammes d'une solution de palmitoyl-pentapeptide-3 à 3 grammes d'une solution de méthylsilanol hydroxyproline aspartate, à 0,1 gramme d'allantoïne et à 1 gramme d'huile d'avocat pour obtenir une composition A suivant l'invention.

On reprend les mêmes opérations que ci-dessus mais sans ajouter de palmitoyl-pentapeptide-3 pour obtenir une composition B et on reprend les mêmes opérations que ci-dessus, mais cette fois-ci sans ajouter de méthylsilanol hydroxyproline aspartate, pour obtenir une composition C.

On applique ces compositions A, B et C respectivement à des groupes de dix personnes ayant subi une radiothérapie.

On constate que seule la composition C donne le résultat souhaité.

## Revendications

1. Composition comprenant un palmitoyl-pentapeptide-3, **caractérisée en ce qu'**elle comprend du méthylsilanol hydroxyproline aspartate.

2. Composition suivant la revendication 1, **caractérisée en ce que** le palmitoyl-pentapeptide-3 est le palmitoyl-Lys-Thr-Thr-Lys-Ser.

3. Composition suivant l'une des revendications précédentes, **caractérisée en ce que** le rapport pondéral en solutions du palmitoyl-pentapeptide-3 au méthylsilanol hydroxyproline aspartate est compris entre 5/95 et 95/5.

4. Composition suivant la revendication 3, **caractérisée en ce que** le palmitoyl-pentapeptide-3 représente de 30 à 50% et le méthylsilanol hydroxyproline aspartate, de manière correspondante, de 70 à 50% du poids total du palmitoyl-pentapeptide-3 et du méthylsilanol hydroxyproline aspartate dans la composition.

5. L'utilisation d'une composition suivant les revendications précédentes pour fabriquer un médicament pour atténuer les effets sur la peau induits par les effets de radiothérapie.

6. L'utilisation d'une composition suivant les revendications 1 à 4 pour fabriquer un médicament pour apaiser les douleurs cutanées, traiter les vergetures, améliorer la réparation cellulaire dans les cicatrices et/ou la sénescence cutanée.

## Claims

1. Composition comprising a palmitoyl-pentapeptide-3, **characterised in that** it comprises methylsilanol hydroxyproline aspartate.

2. Composition according to claim 1, **characterised in that** the palmitoyl-pentapeptide-3 is palmitoyl-Lys-Thr-Thr-Lys-Ser.

3. Composition according to one of the preceding claims, **characterised in that** the ratio by weight in solution of the palmitoyl-pentapeptide-3 to the methylsilanol hydroxyproline aspartate is between 5/95 and 95/5.

4. Composition according to claim 3, **characterised in that** the palmitoyl-pentapeptide-3 represents from 30 to 50% and the methylsilanol hydroxyproline aspartate correspondingly represents from 70 to 50% of the total weight of the palmitoyl-pentapeptide-3 and the methylsilanol hydroxyproline aspartate in the composition.

5. Use of a composition according to the preceding claims for producing a medicament for attenuating the effects on the skin induced by the effects of radiotherapy.

6. Use of a composition according to claims 1 to 4 for producing a medicament for soothing skin pains, treating stretch marks, improving cell repair in scars and/or skin senescence.

## Patentansprüche

1. Zusammensetzung, enthaltend ein Palmitoylpentapeptid-3, **dadurch gekennzeichnet, dass** sie Methylsilanol-Hydroxyprolin-Aspartat enthält.

2. Zusammensetzung nach Anspruch 1, **dadurch gekennzeichnet, dass** das Palmitoylpentapeptid-3 das Palmitoyl-Lys-Thr-Thr-Lys-Ser ist.

3. Zusammensetzung nach einem der vorhergehenden Ansprüche, **dadurch gekennzeichnet, dass** das Lösungsgewichtsverhältnis von Palmitoylpentapeptid-3 zu Methylsilanol-Hydroxyprolin-Aspartat zwischen 5/95 und 95/5 liegt.

4. Zusammensetzung nach Anspruch 3, **dadurch gekennzeichnet, dass** das Palmitoylpentapeptid-3 30 bis 50 % und das Methylsilanol-Hydroxyprolin-Aspartat dementsprechend 70 bis 50 % des Gesamtgewichts von Palmitoylpentapeptid-3 und Methylsilanol-Hydroxyprolin-Aspartat in der Zusammensetzung ausmacht.

5. Verwendung einer Zusammensetzung nach den vorhergehenden Ansprüchen zur Herstellung eines Medikaments zur Milderung der Auswirkungen von Strahlentherapie auf die Haut.

6. Verwendung einer Zusammensetzung nach den Ansprüchen 1 bis 4 zur Herstellung eines Medikaments zur Reduzierung von Hautschmerzen, Behandlung von Schwangerschaftsstreifen, Besserung der Zellreparatur bei Narben und/oder bei Hautalterung.
